# EUROPEAN PATENT SPECIFICATION

(11) **EP 1 641 420 B1**
(45) Date of publication and mention of the grant of the patent: **27.02.2013**
(21) Application number: 04743091.3
(22) Date of filing: 24.06.2004
(51) Int. Cl.: A61G 7/10, A61F 15/00

(54) **MEDICAL PROTECTION SHEETING**
MEDIZINISCHE SCHUTZHÜLLE
TOILE DE PROTECTION MEDICALE

(30) Priority: 24.06.2003 GB 0314659
(43) Date of publication of application: 05.04.2006
(73) Proprietor: APA PARAFRICTA LTD., Buckinghamshire MK16 8NR (GB)
(72) Inventor: PIKE, Anthony Bruce, Upper Norwood, London SE19 2DN (GB)
(74) Representative: Wise, Stephen James
(86) International application number: PCT/GB2004/002740
(87) International publication number: WO 2005/000183

(56) References cited:
- EP-A- 0 469 767
- EP-A- 0 966 892
- GB-A- 2 338 700
- US-A- 3 648 291
- US-A- 4 051 565

## Description

The present invention relates to an article comprising a medical protection sheeting useful for patient handling and designed to reduce the risk of damage to the skin of patients where this is damaged or where it is subject to pressure.

Patient handling sheeting is known for turning patients or lifting them between beds and trolleys and are sometimes known as "slide sheets", a term which will be used below. Where patients are severely injured, the movement between beds and trolleys can be difficult with known slide sheets since the material used for slide sheets is such as to cause high initial resistance to sliding, that is, the coefficient of friction rapidly rises as the sheet is pulled, reaching a peak before sliding actually commences. The sheet then, so to speak, becomes "unstuck". This sudden release from a high frictional resistance is the frequent cause of damage to a patient's skin, particularly where this has been burnt or in other cases where the epidermis is damaged. In some cases, this can cause separation of the dermal-epidermal junction.

Even where the epidermis is not initially damaged, decubitus ulcers or bedsores can form due to reduced blood flow in a local area of skin slowing because of pressure. By frequently changing body positions, this problem can be solved.

Further problems occur with dressings which rub on bedding or clothing. Again, high frictional coefficient between the dressing and material with which it is in contact can cause rucking of dressings and again epidermal damage.

GB 2 338 700 discloses an article comprising a patient handling sheeting for transfering a patient.

According to the invention, there is provided an article comprising medical protection sheeting, as claimed in claim 1

The advantage of such a material is that as the material just starts to move over an adjacent material, which where possible is the same material, there is a smooth and gentle initiation and acceleration with no jerking. Thus epidermal damage is limited. Where slide sheets are made of the material of the invention, there is also less energy required by those caring for the patient. Where there is a concern for bedsores, then the patient may himself be able to move alternatively and again less energy may be used by carers to frequently move the patient lying on the material.

The material is a woven fabric to enable a certain amount of "breathing". The material should not be too coarse and it is considered that materials having a linear density of 1000 to 40 decitex should be satisfactory. Materials of 470, 350 and 50 decitex have been tried and perform well. The materials weigh respectively for the 350 and 50 decitex material 180 and 61.7 gm/m². The finest material used which performed well under test was DuPont Tactel (RTM) having nylon warp yarn 50F15T143 and nylon weft yarn 50F15T1943. A coarser fabric identified as 470T743 has been used with success. A medium weight fabric identified as DuPont's 350T749 was successfully used.

Embodiments of the invention will now be described by way of example with reference to the accompanying drawings and diagrams in which:-
Figure 1 is a diagram of a test rig to test materials for the invention;
Figure 2 shows test results from the rig of Figure 1 to compare material according to the invention with linen so as to indicate relative coefficients of friction;
Figure 3 is an extract from the results of Figure 2;
Figure 4 is a set of test results from the rig of Figure 1 showing how varying the relative orientation between samples of the same material according to the invention varies the friction between the samples; and
Figure 5 shows a bootee made with material according to the invention.

Samples of DuPont (DTI) plain woven nylon fabric from which slide sheets and dressing covering were made and were tried out as follows:-

| **Parafricta (TM) Ref** | **DTI Ref** | **Linear Density (decitex)** | **Weight g/m²** | **Gauge mm** | **Finished Threads per cm** | **Tensile Strength kN** |
|---|---|---|---|---|---|---|
| P470 | 470T743 | 470 | | 0.4 | 24 x 20 | |
| P350 | 350T749 | 350 | 180 | <0.35 | | >2.3 |
| P50 | 98Wext/4 | 50 | 61.7 | 0.15 | 61x47 | 6* |

| | | | | | | |
|---|---|---|---|---|---|---|
| *Shear strength kN/m² | | | | | | |

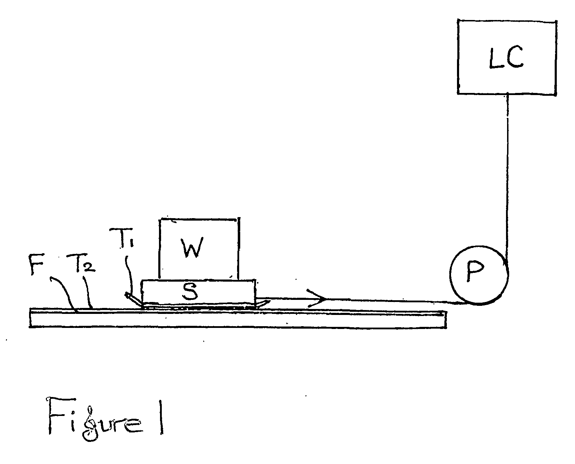

Samples of P470 fabric were submitted to the test rig shown in Figure 1 for the purpose of comparing its performance with linen on a linen to linen basis, P470 to P470, P470 to linen and linen to P470.

A block on flat measurement technique was used (Figure 1). A 50 x 50 mm sledge S wrapped in test fabric T1 was pulled over a flat plate F which was covered by a sheet of the second fabric T2 to be tested. The sledge was pulled at a constant speed of 1 mm per second using a steel wire and a pulley by an Instron mechanical testing system LC. The load that was needed to pull the sledge gives the friction load and was recorded throughout the test. The sledge was loaded by weights giving a 40 N vertical load during the test. The friction coefficient is calculated as the frictional force divided by the applied load.

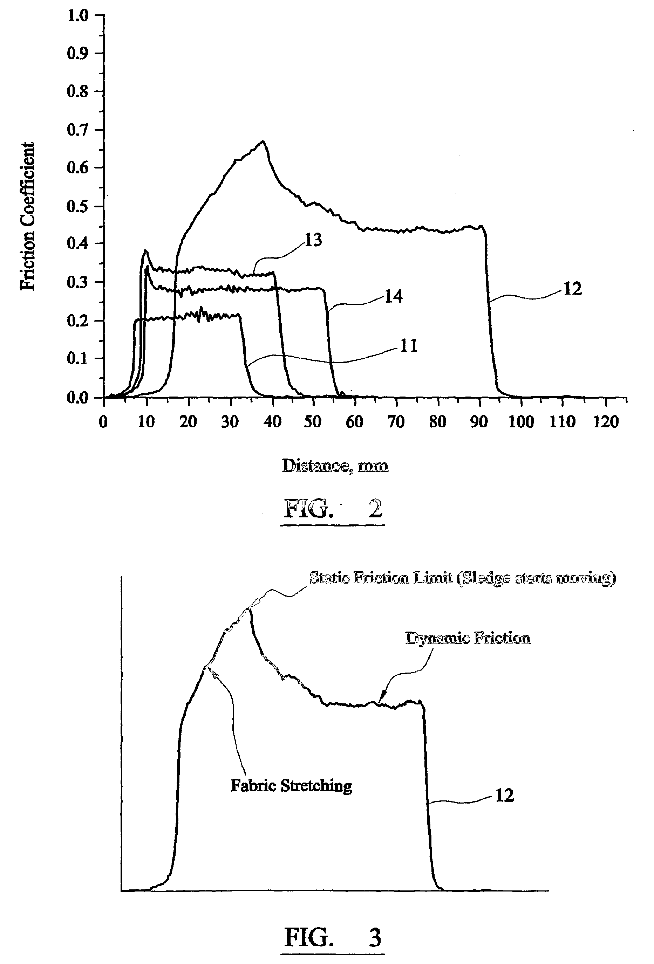

The results are shown schematically in Figure 2, with the main features given in Table 1. The highest friction values were recorded for the linen rubbing against linen where the static friction coefficient was 0.67 and the dynamic friction coefficient was 0.44. By contrast the static friction coefficient and dynamic friction coefficient were about 0.2 for the P470 rubbing on itself. When P470 was rubbing on linen, or linen on P470, the results were very similar with a dynamic friction coefficient of about 0.3 with a slightly higher static friction coefficient. Figure 3 extracted from Figure 2 shows that static friction increases rapidly from the application of a load until linen stretches. The linen then suddenly starts to slide, at which point the dynamic friction decreases rapidly.

**Table 1, Main Results**

| **Figure 2** **Trace** | **Sledge Fabric (T1)** | **Flat Fabric (T2)** | **static Friction** | **Dynamic Friction** |
|---|---|---|---|---|
| 12 | Linen | Linen | 0.67 | 0.44 |
| 11 | P470 | P470 | 0.19 | 0.21 |
| 13 | P470 | Linen | 0.38 | 0.33 |
| 14 | Linen | P470 | 0.33 | 0.29 |

Having completed the comparative tests of P470 against linen, a second series of tests was conducted to determine the effect of relative orientation as between warp and weft of P470 material. The same rig (Figure 1) was used as in the first series of tests.

In the first set of the second series of tests, the load was recorded by chart recorder and then scanned and digitised to obtain digital results. For the second set of measurements, the load was recorded directly by a data logging system in a digital form.

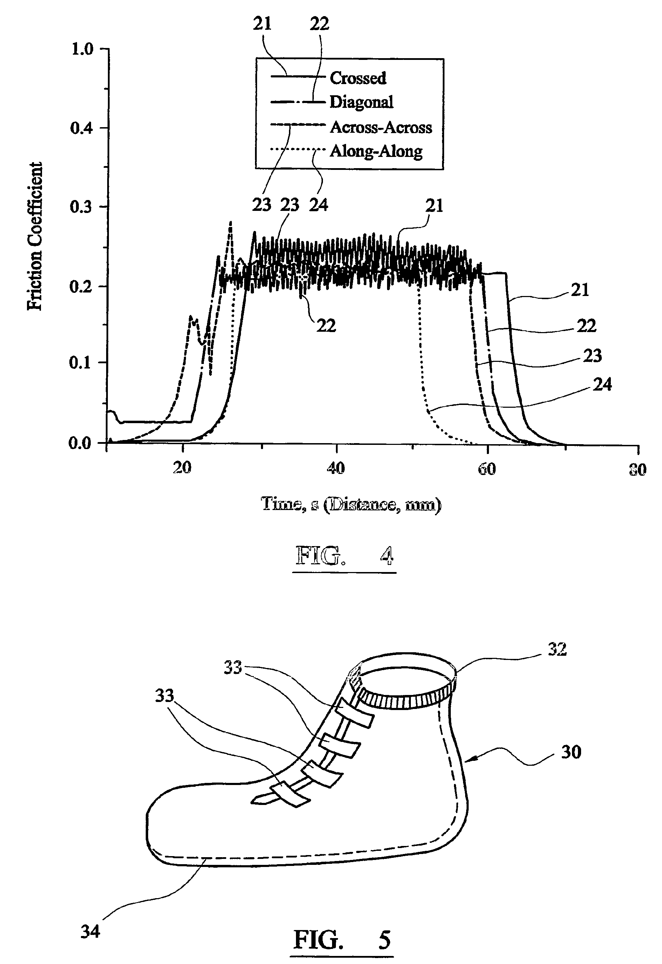

The results are shown schematically in Figure 4, with the main features given in Table 2. The highest friction values were recorded for the linen rubbing against linen where the static friction coefficient was 0.67 and the dynamic friction coefficient was 0.44. By contrast, the static friction coefficient and dynamic friction coefficient were about 0.2 for the P470 rubbing on itself. When P470 was rubbing on linen, or linen on P470, the results were very similar, with a dynamic friction coefficient of about 0.3 with a slightly higher static friction coefficient. There was very little difference between the friction results for all the P470 on P470 tests.

The uncertainty of measurements of this type has not been studied definitively, but general experience of friction measurements would suggest that at an average friction level of about 0.2, the uncertainty in friction coefficient measurement is about 0.03.

**Table 2, Further Results. Across is the direction on the P470 fabric parallel to the weft on the sample supplied. Along is perpendicular to this and parallel to the warp.**

| **Figure 4** **Trace** | **Sledge Fabric T1** | **Flat Fabric T2** | **Static Friction** | **Dynamic Friction** |
|---|---|---|---|---|
| | Linen | Linen | 0.67 | 0.44 |
| 24 | P470 - Along | P470 - Along | 0.19 | 0.21 |
| | P470 - Along | Linen | 0.38 | 0.33 |
| | Linen | P470 - Along | 0.33 | 0.29 |
| 21 | P470 - Along | P470 - Across | 0.24 | 0.24 |
| 22 | P470 - Diagonal | P470 - Across | 0.21 | 0.21 |
| 23 | P470 - Across | P470 - Across | 0.23 | 0.23 |

Trace 21 marked "crossed" on Figure 4 indicates the Sledge and Flat fabrics are orientated warp to weft or Along/Across.

The comparative second series of tests showed that the P470 material was best aligned to another sample of itself so that both the warp threads were parallel, that the movable sheet was pulled in the warp direction and remarkably that the static friction was slightly less than the dynamic friction. The most unfavourable relative orientation was when the P470 materials were warp to weft or "crossed". However, as long as P470 was arranged to slide on P470, there was no great difference in the coefficient of friction for any relative orientation. In none of the relative orientations was there any great difference and most had the same static and dynamic coefficient of friction. This showed that unlike the linen on linen case shown in Figure 3, there was no tendency for sudden jerking when using P470 to P470 in juxtaposition. Sudden jerking would result in sudden uncontrolled motion.

Although it is desirable to use P470 to P470, the results of the tests of P470 to linen show that this is considerably better than using linen to linen. Furthermore, when using P470 to linen, it is best to pull the P470 in the warp direction when on linen. It is therefore preferable when making a slip sheet (often about 120 x 70 cm or 145 x 72 cm) to cut the material so that the long side of the sheet runs parallel with the weft. Obviously, a range of sizes may be supplied up to about 200 cm long and up to 100 cm wide.

It will be observed that when using the material of the invention as a slide sheet, the force F to pull the sheet under a patient of weight W, the force required where µ is the coefficient of friction and g is the acceleration due to gravity, that F = 2µgW, where static µ (µs) is much greater than dynamic µd, then F is increasingly greater to start moving the patient. Generally it can be shown that the material of the invention requires only about half the effort (F) when compared to a sliding linen over linen. Also, the material can be made strong for any given weight of material.
Generally, µs should be no more than 20% greater than µd and both should be less than 0.4.

When using the material of the invention as a covering for a dressing, for example, as shown in Figure 5 which is in the form of a bootee 30 with a Lycra (RTM) collar 32, adjustable Velcro (RTM) closers 33 and externally seamed 34, a finer material such as P50 is preferred. Other forms (not shown) of covering for dressings are provided but which are also shaped to other particular parts of the body. The bootee can have its toe cut off to allow ventilation.

A particular application of the covering according to the invention is to protect dressings comprising advanced materials such as hydrocolloids which could include active materials to protect against infection or promote healing. Although these dressings may be more expensive than conventional dressings, the protection given by the covering of the invention enables these advanced dressings to remain in place longer. Therefore cost savings ensue.

## Claims

1. An article comprising a medical protection sheeting useful for patient handling formed from woven material **characterised in that** the material has a coefficient of static friction between itself and linen of less than 0.4 and that the coefficient of static friction of the material with itself is substantially the same as its coefficient of dynamic friction with itself, wherein the sheeting includes a patient contacting surface.

2. An article as claimed in claim 1 wherein the material is woven from a yarn having a linear density between 1000 and 40 decitex.

3. An article as claimed in claim 2 wherein the linear density is 470, 350 or 50 decitex.

4. An article as claimed in claim 2 or 3 wherein the weight of the material is between 200 and 50 gm/m².

5. An article as claimed in claim 2 wherein the material's weight is 180 gm/m² for the 350 decitex material and about 62 gm/m² for the 50 decitex material.

6. An article as claimed in any one of claims 1 to 5, wherein the sheeting is formed as a bootee (30) with one or more layers of the material.

7. An article as claimed in claim 6 wherein the bootee (30) is formed without a toe.

8. An article as claimed in any one of claims 1 to 5 wherein the sheeting is formed as a dressing.

## Patentansprüche

1. Ein Artikel, der eine für den Umgang mit Patienten nützliche, aus gewebtem Material gebildete medizinische Schutzmatte beinhaltet, **dadurch gekennzeichnet, dass** das Material einen Koeffizienten einer Haftreibung zwischen sich und Bettwäsche von weniger als 0,4 aufweist und dass der Koeffizient einer Haftreibung des Materials mit sich im Wesentlichen der gleiche ist wie sein Koeffizient einer Gleitreibung mit sich, wobei die Matte eine Patientenberührungsoberfläche umfasst.

2. Artikel gemäß Anspruch 1, wobei das Material aus einem Garn, das eine Feinheit zwischen 1000 und 40 Dezitex aufweist, gewebt ist.

3. Artikel gemäß Anspruch 2, wobei die Feinheit 470, 350 oder 50 Dezitex beträgt.

4. Artikel gemäß Anspruch 2 oder 3, wobei das Gewicht des Materials zwischen 200 und 50 g/m² beträgt.

5. Artikel gemäß Anspruch 2, wobei das Gewicht des Materials 180 g/m² für das 350-Dezitex-Material und etwa 62 g/m² für das 50-Dezitex-Material beträgt.

6. Artikel gemäß einem der Ansprüche 1 bis 5, wobei die Matte als Schuh (30) mit einer oder mehreren Schichten des Materials gebildet wird.

7. Artikel gemäß Anspruch 6, wobei der Schuh (30) ohne Fußspitze gebildet wird.

8. Artikel gemäß einem der Ansprüche 1 bis 5, wobei die Matte als Verband gebildet wird.

## Revendications

1. Un article comprenant une toile de protection médicale utile pour la manipulation des patients formée à partir de tissu tissé **caractérisé en ce que** le tissu a un coefficient de frottement statique entre lui-même et du linge inférieur à 0,4 et **en ce que** le coefficient de frottement statique du tissu avec lui-même est substantiellement le même que son coefficient de frottement dynamique avec lui-même, dans lequel la toile inclut une surface de contact avec le patient.

2. Un article tel que revendiqué dans la revendication 1 dans lequel le tissu est tissé à partir d'un fil ayant une masse linéaire comprise entre 1 000 et 40 décitex.

3. Un article tel que revendiqué dans la revendication 2 dans lequel la masse linéaire est de 470, 350 ou 50 décitex.

4. Un article tel que revendiqué dans la revendication 2 ou la revendication 3 dans lequel la masse du tissu est comprise entre 200 et 50 g/m².

5. Un article tel que revendiqué dans la revendication 2 dans lequel la masse du tissu est de 180 g/m² pour le tissu de 350 décitex et d'environ 62 g/m² pour le tissu de 50 décitex.

6. Un article tel que revendiqué dans une quelconque des revendications 1 à 5, dans lequel un chausson (30) est formé avec la toile à l'aide d'une ou de plusieurs couches du tissu.

7. Un article tel que revendiqué dans la revendication 6 dans lequel le chausson (30) est formé sans bout.

8. Un article tel que revendiqué dans une quelconque des revendications 1 à 5 dans lequel un pansement est formé avec la toile.
